## Europäisches Patentamt
## European Patent Office
## Office européen des brevets

(11) Publication number: **0 084 334**
**B1**

(12) **EUROPEAN PATENT SPECIFICATION**

(45) Date of publication of patent specification: **14.05.86**

(51) Int. Cl.⁴: **C 12 P 19/26, A 61 K 31/73 //**
**C08B37/00**

(21) Application number: **83100112.8**

(22) Date of filing: **08.01.83**

(54) **Polysaccharide substance, process for the production of same, pharmaceutical compositions containing the same and their use as medicaments.**

(30) Priority: **14.01.82 JP 3411/82**

(43) Date of publication of application:
**27.07.83 Bulletin 83/30**

(45) Publication of the grant of the patent:
**14.05.86 Bulletin 86/20**

(84) Designated Contracting States:
**AT BE CH DE FR GB IT LI NL SE**

(56) References cited:
**CHEMICAL ABSTRACTS, vol. 90, no. 7, 12th February 1979, page 297, no. 51201g, Columbus Ohio (USA); R.A. MacLEOD et al.: "Translocation of lipopolysaccharide in the cell wall of a gram-negative marine bacterium"**

**CHEMICAL ABSTRACTS, vol. 89, no. 25, 18th December 1978, page 281, no. 211626j, Columbus Ohio (USA); J.M. DiRIENZO et al.: "Composition of the fractions separated by polyacrylamide gel electrophoresis of the lipopolysaccharide of a marine bacterium"**

(73) Proprietor: **MICROBIAL CHEMISTRY RESEARCH FOUNDATION**
**14-23, Kamiosaki 3 Chome Shinagawa-ku Tokyo 141 (JP)**

(72) Inventor: **Umezawa, Hamao, Prof.**
**4 chome-23, Toyotama-kita Nerima-ku Tokyo (JP)**
Inventor: **Okami, Yoshiro**
**4-18-14, Denenchofu Ohta-ku Tokyo (JP)**
Inventor: **Takeuchi, Tomio**
**5-1-11, Higashi-Gotanda Shinagawa-ku Tokyo (JP)**
Inventor: **Kurasawa, Shogo**
**5-39-2, Sendagi Bunkyo-ku Tokyo (JP)**
Inventor: **Yamada, Kazuhiko**
**3-22-22, Fujigaoka Fujisawa-city Kanagawa Prefecture (JP)**
Inventor: **Suzuki, Katsumi**
**3-1631, Ikebukuro Toshima-ku Tokyo (JP)**
Inventor: **Shiio, Tsuyoshi**
**1495-5, Yamazaki Kamakura-city Kanagawa prefecture (JP)**

Courier Press, Leamington Spa, England.

**0 084 334**

(56) References cited:
CHEMICAL ABSTRACTS, vol. 89, no. 25, 18th December 1978, page 281, no. 211625h, Columbus Ohio (USA); J.M. DiREINZO et al.: "Hetereogeneity and distribution of lipopolysaccharide in the cell wall of a gram-negative marine bacterium"

CHEMICAL ABSTRACTS, vol. 86, no. 17, 25th April 1977, page 229, no. 117350f, Columbus Ohio (USA); J.D. BELSON Jr. et al.: "Distribution of lipopolysaccharide and the detection of a new subfraction in the cell envelope of a marine pseudomonad"

CHEMICAL ABSTRACTS, vol. 91, no. 7, 13th August 1979, page 323, no. 52415n, Columbus Ohio (USA); K.-Y. CAHN et al.: "Requirement of sodium by marine heterotrophic bacteria"

CHEMICAL ABSTRACTS, vol. 80, no. 17, 29th April 1974, page 178, no. 93051g, Columbus Ohio (USA); L. BAUMANN et al.: "Regulation of aspartokinase activity in nonfermentative, marine eubacteria"

CHEMICAL ABSTRACTS, vol. 86, no. 1, 3rd January 1977, page 192, no. 2073a, Columbus Ohio (USA); M.J. GAUTHIER et al.: "Antibacterial activity of marine violet-pigmented alteromonas with special reference to the production of brominated compounds"

CHEMICAL ABSTRACTS, vol. 88, no. 5, 30th January 1978, page 230, no. 34276b, Columbus Ohio (USA); M.J. GAUTHIER: "Alteromonas citrea, a new gram-negative, yellow-pigmented species from seawater"

CHEMICAL ABSTRACTS, vol. 96, no. 21, 24th May 1982, page 389, no. 177631q, Columbus Ohio (USA); R.A. LADDAGA et al.: "Effects of wash treatments on the ultrastructure and lysozyme penetrability of the outer membrane of various marine and two terrestrial gram-negative bacteria"

JOURNAL OF BACTERIOLOGY, vol. 110, no. 1, April 1972, pages 402-429, (USA); L. BAUMANN et al.: "Taxonomy of aerobic marine eubacteria"

⑦② Inventor: Ishizuka, Masaaki
3-17, Denenchofu-Honcho Ohta-ku
Tokyo (JP)
Inventor: Ohnuki, Takashi
1155-2, Nakamaruko Nakahara-ku
Kawasai-city Kanagawa Prefecture (JP)

⑦④ Representative: Reuter, Johann-Heinrich, Dr. et al
HOECHST AKTIENGESELLSCHAFT Zentrale
Patentabteilung Postfach 80 03 20
D-6230 Frankfurt/Main 80 (DE)

**Description**

This invention relates to the polysaccharide substance MP-86, and to a process for the production of the same and their use as medicaments.

A number of polysaccharides have heretofore been discovered, and it is known that they have a wide variety of uses. It has now been found that a specific micro-organism produces the novel polysaccharide substance MP-86, and this substance has antitumour activity and immunopotentiating activity.

The polysaccharide substance MP-86 of this invention shows the following analysis and properties:

a. A carbon to nitrogen ratio, determined by elemental analysis, of 36:1, although accurate analysis is difficult because said substance is hygroscopic;

b. An average molecular weight, determined by gel filtration using Sepharose CL-4B®, of $5\times10^4$ to $15\times10^4$;

c. Insoluble in organic solvents and soluble in water;

d. Positive in a color development test by the phenol-sulfuric acid method;

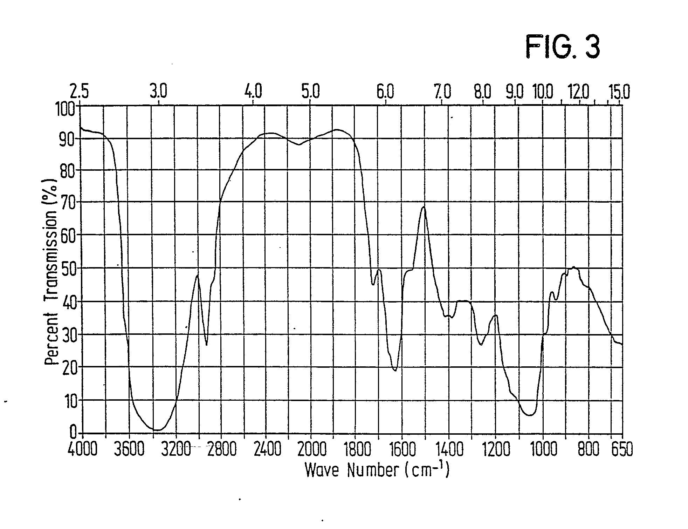

e. The sugar composition determined by gas chromatography: 25±3% glucose, 25±3% mannose, 21±2% galactose, 14±1% rhamnose, and 5.0±0.5% fucose; and

f. An infrared absorption spectrum as shown in Figure 3.

The polysaccharide substance MP-86 is present in the fermentation broth of microorganisms belonging to the genus Alteromonas and can be obtained therefrom. The polysaccharide substance MP-86 of this invention can be produced, for example, by the cultivation of a microorganism belonging to the genus Alteromonas having the ability to produce the polysaccharide substance MP-86 in a medium consisting of carbon sources, nitrogen sources, inorganic ions, and if necessary, organic nutrients such as vitamins and amino acids, dissolved in seawater (including artificial seawater). The polysaccharide substance MP-86 produced and accumulated in the fermentation broth is separated and recovered.

Any carbon sources which are commonly used for the cultivation of microorganisms can be used in the present invention. Preferred examples of such carbon sources are carbohydrates such as glucose, sucrose, starch and dextrin. Also, nitrogen sources which are commonly used for the cultivation of microorganisms can be used in the present invention. Examples of such nitrogen sources include peptone, yeast extract, meat extract, corn steep liquor, soybean powder, casein and ammonium ions.

Cultivation is performed preferably under aeration with stirring. The cultivation temperature can be appropriately selected within a range which permits the MP-86-producing microorganism to grow well and produce the polysaccharide substance MP-86. The usual range is preferably from 10 to 35°C, more preferably from 24 to 30°C. The cultivation is performed until a sufficient amount of the desired polysaccharide substance MP-86 is accumulated in the fermentation broth. Usually, the cultivation time is from 15 to 100 hours.

An example of microorganisms capable of producing the polysaccharide substance MP-86 of the present invention is *Alteromonas vaga* MP-86 (FERM P-6282).

This strain was collected and isolated from marine environment and has the following microbiological characteristics:

a) Morphological characteristics
1. Form and size of the cell:
   Rod to spherical, 0.3 to 0.5×1.0 to 1.5 µm
2. Pleomorphism: Non-pleomorphic
3. Motility/flagellation:Motile/polar flagella
4. Sporogenicity: Non-sporulating
5. Gram's stain: Negative
6. Acid-fastness: Negative

b) Culture characteristics on various media
1. Bouillon agar plate culture: No growth
1′. Marine agar 2216 medium plate culture:
   Medium growth, circular, semilenticular, entire margon, smooth, glistening, transparent, butyraceous, and pale yellow
2. Bouillon agar slant culture: No growth
2′. Marine agar slant culture: Medium growth, membranous, thread-like, glistening, and faint yellow
3. Bouillon liquid culture: No growth
3′. Marine medium liquid culture: Uniformly turbid, no membrane formation, and powder precipitation
4. Bouillon gelatin stab culture: No changes
4′. Marin 2216 medium gelatin stab culture: No liquefaction
5. Litmus milk: No changes

3

0 084 334

c) Physiological characteristics
1. Reduction of nitrates: Negative
2. Denitrification: Negative
3. MR test: Negative
4. VP test: Negative
5. Production of indole: Negative
6. Production of hydrogen sulfide: Negative
7. Hydrolysis of starch: Negative
8. Utilization of citric acid: Negative on a Koser medium, and negative on a Christensen medium.
9. Utilization of inorganic nitrogen source: Utilizes nitrates and ammonium salts.
10. Production of pigment: Negative
11. Urease: Positive
12. Oxidase: Negative
13. Catalase: Positive
14. Growth ranges:
Temperature: growth below 35°C and no growth at 40°C pH: 6 to 9
15. Oxygen demand: Aerobic
16 O—F test (according to the Hugh & Leifson method: No acid is formed.
17. Production of acids and gases from saccharides (culture composition: 0.5% peptone, 2% NaCl, 1% $MgCl_2$, 0.2% $CaCl_2$, 0.1% KCl and 0.02% BCP):

| Saccharides | Production of acid | Production of gas |
|---|---|---|
| L-Arabinose | − | − |
| D-Xylose | − | − |
| D-Glucose | + | − |
| D-Mannose | + | − |
| D-Fructose | − | − |
| D-Galactose | − | − |
| Maltose | − | − |
| Sucrose | − | − |
| Lactose | − | − |
| Trehalose | − | − |
| D-Sorbitol | − | − |
| D-Mannitol | − | − |
| Inositol | − | − |
| Glycerin | ± | − |
| Starch | − | − |
| Raffinose | − | |
| Rhamnose | − | |

18. Arginine dihydrolase reaction (according to the method of R. Y. Stanier et al., J. Gen. Microbiol. 43, 159 (1966):Negative
19. Decomposition of casein: Negative
20. Accumulation of poly-β-hydroxybutyric acid: Negative
21. Nutrient requirement: No growth occurs unless seawater is added.

4

22. Utilization of the following compounds
(Stanier medium+Marine salts)

| | | | | |
|---|---|---|---|---|
| D-Glucose | + | Butyric acid | − |
| L-Arabinose | − | m-Benzoic acid | − |
| D-Xylose | + | p-Benzoic acid | − |
| D-Mannose | + | meso-Tartaric acid | − |
| D-Galactose | + | Levulinic acid | − |
| Saccharose | − | Citraconic acid | − |
| Lactose | − | Gluconic acid | + |
| Cellobiose | + | Malonic acid | − |
| Salicin | − | L-Alanine | + |
| Erythritol | + | β-Alanine | − |
| D-Mannitol | + | L-Valine | − |
| Glycerin | + | L-Ornithine | + |
| D-Fructose | + | L-Lysine | − |
| Inositol | − | L-Tyrosine | − |
| Trehalose | − | DL-Arginine | + |
| Acetic acid | + | Betaine | + |
| Succinic acid | + | Ethanol | − |
| Citric acid | + | DL- -Hydroxybutyrate | + |
| Lactic acid | + | Fumaric acid | + |
| Propionic acid | − | α-Ketoglutaric acid | + |

23. GC content of DNA: 47.0%

In view of the above microbiological characteristics, Bergey's Manual of Determinative Bacteriology, 8th Ed. has been referred to for the identification of the strain used in the present invention. This strain does not agree with the genus Pseudomonas having a DNA-GC content of 58 to 70%. Vibrio, Photobacterium, Beneckea and Lucibacterium species are inconsistent with the strain because these species are facultative anaerobes and the other characteristics of them are also different from those of the strain. Acinetobacter and Moraxella species, non-motile bacteria, differ from the strain. Bdelloxibrio species, obligatory parasites, do not conform to the strain. Halobacterium species, having a DNA-GC content of 57 to 68%, do not agree with the strain. The strain of the invention thus differs from the genera described in said manual in terms of important characteristics and does not conform to any of the genera.

Search through taxonomic systems other than those revealed in said manual has shown the strain of the invention to be in good agreement with the genus Alteromonas (Gram-negative, rod, non-fermentative, polar flagellate, DNA-GC content of 40 to 50%, marine bacteria, aerobic) proposed by Baumann et al. (Taxonomy of aerobic marine eubacteria, J. Bacteriol. *110*, 402—429, 1972). This strain has therefore been identified as a bacterium belonging to the genus Alteromonas. Among the known species of this genus, *Alteromonas vaga* (Baumann et al., 1972) is in accordance with the strain of the present invention possessing the following characteristics: a DNA-GC content of 47.0%, negativeness of oxidase, no growth at 40°C, and no decomposition of starch. The present strain has been deposited in the Fermentation Research Institute, Agency of Industrial Science and Technology, Ministry of International Trade and Industry of Japan under the accession number of FERM P-6282. Any mutants obtained either by such artificial ways as irradiation with ultraviolet rays or with X-rays, treatment with chemicals, or spontaneous mutation can be used in the present invention as long as they have the ability to produce the

5

polysaccharide substance MP-86 of the present invention. The polysaccharide substance MP-86 of this invention can be recovered from the fermentation broth by known methods. For example, the fermentation broth is subjected to centrifugal separation at 7,500 rpm for fifteen minutes to provide a supernatant liquid. A lower alkanol, preferably ethanol, is added to the supernatant liquid, and the resulting mixture is allowed to stand in an ice chamber to cause precipitation. The precipitate is isolated, and dried under reduced pressure to obtain a crude form of the polysaccharide substance MP-86.

The crude polysaccharide substance MP-86 thus obtained is then further purified. For this purpose, various methods can be used. For example, the crude substance is dissolved in a buffer solution, and treated with protease. Then the solution is passed through a Sepharose CL-4B® column, and eluted with 0.05 N-NaCl at a suitable flow rate. The eluate is fractionated in predetermined amounts, and MP-86-containing fractions are collected and dialyzed to remove NaCl. Then, ethanol is added to cause precipitation whereby a purified MP-86 fraction can be obtained.

The physical and chemical properties of the polysaccharide substance MP-86 of this invention are described below.

0.5 ml of 72% sulfuric acid was added to 10 mg of the polysaccharide substance MP-86, and the mixture was allowed to stand for two hours at room temperature. To the mixture was added 7 ml of water, and the resulting mixture was placed in a sealed tube. The tube was heated in an oil bath at 105°C for two hours. The reaction mixture was neutralized with barium hydroxide, and the resulting barium sulfate was removed by filtration. The filtrate was dried under reduced pressure, and trimethylsilylated with a commercially available trimethylsilylating agent. Gas chromatographic analysis with reference to a standard sample shows that the polysaccharide substance MP-86 of this invention has the composition given in Table 1.

TABLE 1

| | |
|---|---|
| Glucose | 25±3% |
| Mannose | 25±3% |
| Galactose | 21±2% |
| Rhamnose | 14±1% |
| Fucose | 5.0±0.5% |

Similar to other polysaccharides, the polysaccharide substance MP-86 of this invention is hygroscopic, and thus difficult to analyze accurately. One example of its elemental analysis is C:38.98%, H:6.45% and N:1.08%.

The carbon/nitrogen ratio of the polysaccharide substance MP-86 of this invention is C:N=36:1.

The average molecular weight determined by a gel filtration method using Sepharose CL-4B® is $5\times10^4$ to $15\times10^4$. The infrared absorption spectrum obtained by the KBr tablet method is shown in Figure 3. The ultraviolet absorption spectrum shows no maximum absorption.

The polysaccharide substance MP-86 of the present invention is white. This substance is insoluble in organic solvents, but soluble in water. The substance is positive in a color development test by the phenol-sulfuric acid method. The components of the polysaccharide substance MP-86 of this invention contain unidentified substances. To identify these substances, further investigation was carried out in the following way: 1 ml of $1N-H_2SO_4$ was added to about 5 mg of Sample III to be described later. The mixture was placed in a sealed tube, and heated in an oil bath at 100°C for five hours for hydrolysis. After cooling, the reaction mixture was neutralized with a saturated solution of barium hydroxide, and centrifuged at 3,000 rpm for fifteen minutes to remove the precipitate formed. The supernatant was lyophilized, and dissolved in 0.5 ml of water. The solution was subjected to thin-layer chromatography on a plate (Kieselgel 60 F, a product of Merck) using a 4:4:2 mixture of isopropanol, acetone and 0.1 M lactic acid as a developing agent. The plate had been dipped in a 0.5 M $NaH_2PO_4$ solution, air-dried, and heated at 105°C for one hour. The thin-layer chromatography gave the results shown in Table 2. The sugars were detected by spraying the plate with an anisaldehyde-sulfuric acid solution, followed by heating the sprayed plate at 105°C.

TABLE 2

| Sugar | Standard sample | | Product of the invention | |
|---|---|---|---|---|
| | Rf | Color | Rf | Color |
| Rhamnose | 0.60 | Yellow | 0.60 | Yellow |
| Fucose | 0.47 | Yellow | 0.47 | Yellow |
| Mannose | 0.36 | Brown | 0.36 | Brown |
| Glucose | 0.30 | Blue | 0.30 | Blue |
| | | | 0.27 | Brown |
| Galactose | 0.23 | Bluish green | 0.23 | Bluish green |
| | | | 0.14 | Yellowish brown |

The substance having an Rf of 0.27 and the substance having an Rf of 0.14 were compared with arabinose, sorbose, glucoheptose, talose, altrose, tagatose, fructose, glucosamine, galactosamine and ribose. However, neither of these substances did not conform to any of these sugars. Of these two substances of unknown structure, the substance having an Rf of 0.14 develops a color when reacted with anisaldehyde-sulfuric acid or ninhydrin. The polysaccharide substance MP-86 of this invention has very strong immunopotentiating activity, and is therefore useful for prevention and treatment of infectious diseases. In addition, this substance exhibits its anticancer effect mediated by immunological response of host.

This invention is described in greater detail below with reference to the following examples.

Example 1

*Alteromonas vaga* MP-86 strain (FERM P-6282) was inoculated in a 30-liter jar fermentor containing 15 liters of a medium (pH 7.2) of artificial seawater (Jamarin S, a product of Jamarin Laboratory) containing 0.6% glucose, 0.5% polypeptone and 0.1% yeast extract, and incubated at 27°C for 22 hours under stirring at 300 rpm and aeration at 1/3 vvm.

The fermentation broth was centrifuged at 7500 rpm for fifteen minutes to remove the bacterial cell and to obtain a supernatant. To the supernatant was added a 1.5-fold volume of ethanol. The mixture was allowed to stand overnight in an ice chamber to cause precipitation. The precipitate thus formed was collected, and dried under reduced pressure to obtain a crude form of the polysaccharide substance MP-86 (hereinafter referred to as Sample I) in a yield of 3.5 g. The crude polysaccharide substance MP-86 had a sugar content, determined by the phenol-sulfuric acid method (standard sample: glucose) of 28.1% and a protein content, determined by the Lowry method (standard sample: bovine serum albumin), of 26.0%.

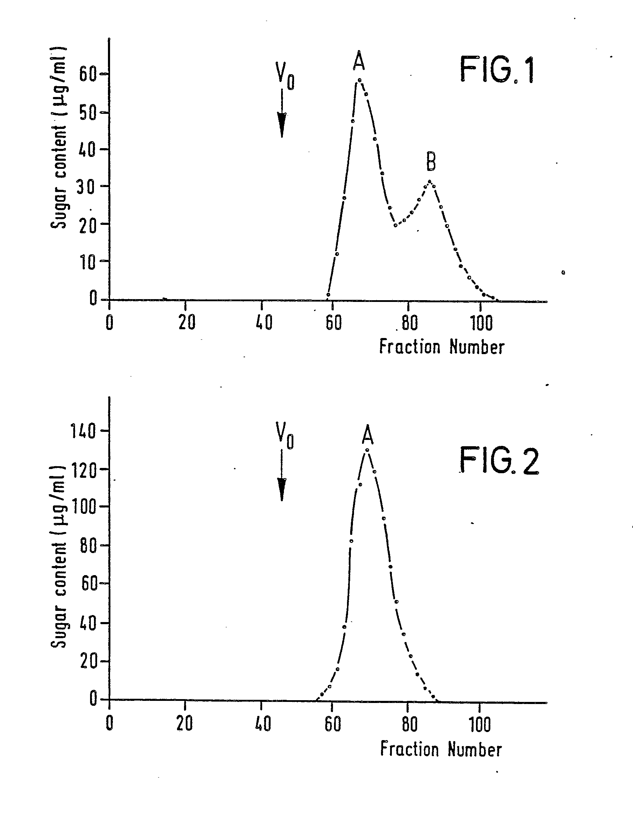

In order to remove protein impurities contained in Sample I, 2 g of Sample I was added to 400 ml of a 40 mM phosphate buffer (pH 7.2), and 4 mg of pronase was further added. The mixture was allowed to stand overnight at 37°C, and then heat-treated with boiling water for fifteen minutes. After cooling, the treated mixture was centrifuged at 10,000 rpm for fifteen minutes to obtain a supernatant. The supernatant was dialyzed overnight against running water to obtain 465 ml of a dialyzate. The dialyzate (hereinafter referred to as Sample II) was analyzed in the same way as for Sample I, and found to have a sugar content of 1.2 mg/ml and a protein content of 0.3 mg/ml. The above dialyzate was concentrated to 90 ml, and each 6 ml, per chromatography, of the concentrate was passed through a Sepharose CL-6B® column (7.5 cm Ø×60 cm, 0.05 N-NaCl), and eluted with 0.05 N-NaCl at a flow rate of 1 ml/min. Figure 1 shows the respective gel filtration chromatogram. The eluate was fractionated into fractions of 20 ml, and Fraction A shown in Figure 1 was collected. Fraction A thus collected was concentrated and dialyzed to obtain 35 ml of a solution containing an active substance. This solution was passed through a Sepharose CL-6B® column to remove Fraction B contained in a trace amount.

Fraction A was collected, and concentrated into 9 ml. The concentrate was dialyzed, and precipitated with the addition of a 2-fold volume of ethanol. The precipitate was dried under reduced pressure to obtain 240 mg of white powder. The same analysis as described above showed the white powder to have a sugar content of 40.6% and a protein content of 0.5%. A 2% solution of this powder sample in deionized water was passed through a Sephadex G-50® column (5 cm o×40 cm) for desalting. The resulting polysaccharide fraction was further concentrated, dialyzed against deionized water, and then lyophilized to obtain white powder of a pure polysaccharide product (hereinafter referred to as Sample III) in a yield of 174 mg. This product had a sugar content of 42.3% and a protein content of less than 0.5%. A chromatogram of Sample III is shown in Figure 2. Sample III had the aforementioned physical and chemical properties.

Example 2

A 3-liter flask was charged with 1 liter of a medium having the same composition as in Example 1. The same strain as shown in Example 1 was inoculated into the medium, and incubated at 27°C for three days under reciprocating shake. Then, the same procedure as used in Example 1 was employed to obtain 183 mg of the crude-form polysaccharide substance MP-86. This substance had a sugar content of 39.5% and a protein content of 24.6%.

Example 3
Antitumor test
a. Sarcoma-180 solid tumor

To ICR mice (five weeks old, female) were subcutaneously transplanted $3 \times 10^6$ Sarcoma-180 tumor cells. For ten days from the day subsequent to the transplantation, a predetermined dose of sample was administered intraperitoneally. At 5th week, the weight of tumor was measured to determine the antitumor activity of the sample. The results are shown in Table 3.

TABLE 3

| Sample | Dose (mg/kg×days) | Number of mice | Tumor inhibition rate (%) | Rate of complete regression* |
|---|---|---|---|---|
| I | 10×10 | 5 | 9.0 | 0/5 |
| | 100×10 | 5 | 98.0 | 2/4 |
| II | 10×10 | 7 | 87.8 | 4/7 |
| III | 50×10 | 7 | 67.9 | 1/7 |

* Number of mice showing complete regression of tumor/number of mice that survived.

b) Sarcoma-180 ascites tumor

To ICR (five weeks old, female) were intraperitoneally transplanted $1 \times 10^6$ Sarcoma-180 tumor cells. For ten days from the day subsequent to the transplantation, a predetermined dose of sample was intraperitoneally administered. The average number of survival days and life-prolonging effect were determined. The results are shown in Table 4.

TABLE 4

| Sample | Dose (mg/kg×days) | Number of mice | Average number of survival days (day) | Increase of life span (%) |
|---|---|---|---|---|
| Control | — | 7 | 15.5± 5.7 | 100.0 |
| I | 25×10 | 7 | 23.0±18.4 | 148.4 |
| II | 25×10 | 7 | 29.1±22.3 | 187.7 |

Action on blastogenesis of spleen cells of mouse

Spleen cells were collected from $CDF_1$ mice (six weeks old, female), and suspended in an RPMI 1640 medium containing 1% of bovine fetal calf serum in a proportion of $1 \times 10^6$ cells per milliliter.

To 0.2 ml of the cell suspension was added 10 microliters of a sample solution, and the mixture was cultured at 37°C in the presence of 5% $CO_2$. Fifty-four hours later, 0.1 uCi of $^3$H-thymidine was added, and cultivation was continued for an additional eighteen hours. Then, the $^3$H-thymidine incorporation to the cultured spleen cells was determined by counting the radioactivity. The results are shown in Table 5.

8

TABLE 5

| Concentration of sample (ug/ml) | Count (cpm/culture) |
|---|---|
| 0 | 355 |
| 0.1 | 348 |
| 0.4 | 355 |
| 1.6 | 482 |
| 6.2 | 963 |
| 25.0 | 1865 |
| 100.0 | 3851 |

**Claims for the Contracting States: Be, CH, DE, FR, GB, IT, LI, NL, SE**

1. A polysaccharide substance having
a) a carbon to nitrogen ratio of 36:1;
b) an average molecular weight of $5 \times 10^4$ to $15 \times 10^4$
c) the sugar composition 25±3% glucose, 25±3% mannose, 21±2% galactose, 14±1% rhamnose, 5.0±0.5% fucose;
d) insolubility in organic solvents and solubility in water;
e) positiveness in a color development test by the phenyl-sulfuric acid method
f) an infrared absorption spectrum as shown in Figure 3, and obtainable by cultivating a microorganism belonging to the genus Alteromonas.

2. A process for producing a polysaccharide substance having
a) a carbon to nitrogen ratio of 36:1;
b) an average molecular weight of $5 \times 10^4$ to $15 \times 10^4$
c) the sugar composition 25±3% glucose, 25±3% mannose, 21±2% galactose, 14±1% rhamnose, 5.0±0.5% fucose,
d) insolubility in organic solvents and solubility in water;
e) positiveness in a color development test by the phenyl-sulfuric acid method
f) an infrared absorption spectrum as shown in Figure 3
which comprises cultivating a microorganism belonging to the genus Alteromonas, and then recovering the polysaccharide product from the culture medium.

3. The process according to Claim 2 wherein a microorganism belonging to the genus Alteromonas is cultivated in a medium consisting of carbon sources, nitrogen sources, inorganic ions and optionally vitamins and amino acids, dissolved in seawater including artificial seawater, under aerated conditions at a temperature of from 10°C to 35°C, and the polysaccharide produced and accumulated in the fermentation broth is separated and recovered.

4. The process according to Claim 3 wherein a microorganism belong to the genus Alteromonas is cultivated at a temperature of from 24°C to 30°C.

5. The process according to Claim 3 wherein the polysaccharide substance is recovered from the fermentation broth by subjecting the latter to centrifugal separation, then adding an alkanol to the supernatant liquid, causing precipitation by cooling and isolating and purifying the precipitate by known methods.

6. The process according to any of Claims 2 to 5 wherein the microorganism used is Alteromonas vaga MP-86 (FERM-P-6282).

7. A pharmaceutical composition comprising as active ingredient a polysaccharide substance as claimed in Claim 1 in association with a pharmaceutically acceptable carrier.

8. A polysaccharide substance as claimed in Claim 1 for use as a medicament.

**Claims for the Contracting State: AT**

1. A process for producing a polysaccharide substance having
a) a carbon to nitrogen ratio of 36:1;
b) an average molecular weight of $5 \times 10^4$ to $15 \times 10^4$
c) the sugar composition 25±3% glucose, 25±3% mannose, 21±2% galactose, 14±1% rhamnose, 5.0±0.5% fucose,
d) insolubility in organic solvents and solubility in water;

e) positiveness in a color development test by the phenyl-sulfuric acid method

f) an infrared absorption spectrum as shown in Figure 3

which comprises cultivating a microorganism belonging to the genus Alteromonas, and then recovering the polysaccharide product from the culture medium.

2. The process according to Claim 1 wherein a microorganism belonging to the genus Alteromonas is cultivated in a medium consisting of carbon sources, nitrogen sources, inorganic ions and optionally vitamins and amino acids, dissolved in seawater including artificial seawater, under aerated conditions at a temperature of from 10°C to 35°C, and the polysaccharide produced and accumulated in the fermentation broth is separated and recovered.

3. The process according to Claim 2 wherein a microorganism belong to the genus Alteromonas is cultivated at a temperature of from 24°C to 30°C.

4. The process according to Claim 2 wherein the polysaccharide substance is recovered from the fermentation broth by subjecting the latter to centrifugal separation, then adding an alkanol to the supernatant liquid, causing precipitation by cooling and isolating and purifying the precipitate by known methods.

5. The process according to any of Claims 1 to 4 wherein the microorganism used is Alteromonas vaga MP-86 (FERM-P-6282).

**Patentansprüche für die Vertragsstaaten: BE, CH, DE, FR, GB, IT, LI, NL, SE**

1. Polysaccharidsubstanz mit

a) einem Kohlenstoff/Stickstoffverhältnis von 36:1,

b) einem Durchschnittsmolekulargewicht von $5 \cdot 10^4$ bis $15 \cdot 10^4$,

c) der Zuckerzusammensetzung 25±3% Glucose, 25±3% Mannose, 21±2% Galaktose, 14±1% Rhamnose und 5,0±0,5% Fucose,

d) Unlöslichkeit in organischen Lösungsmitteln und Löslichkeit in Wasser,

e) positivem Farbentwicklungstest nach der Phenolschwefelsäuremethode und

f) einem Infrarotabsorptionsspektrum wie in Abbildung 3, erhältlich durch Kultivierung eines zur Gattung Alteromonas gehörenden Mikroorganismus.

2. Verfahren zur Herstellung einer Polysaccharidsubstanz mit

a) einem Kohlenstoff/Stickstoffverhältnis von 36:1,

b) einem Durchschnittsmolekulargewicht von $5 \cdot 10^4$ bis $15 \cdot 10^4$,

c) der Zuckerzusammensetzung 25±3% Glucose, 25±3% Mannose, 21±2% Galaktose, 14±1% Rhamnose und 5,0±0,5% Fucose,

d) Unlöslichkeit in organischen Lösungsmitteln und Löslichkeit in Wasser,

e) positivem Farbentwicklungstest nach der Phenolschwefelsäuremethode und

f) einem Infrarotabsorptionsspektrum wie in Abbildung 3, dadurch gekennzeichnet, dass man einen zur Gattung Alteromonas gehörenden Mikroorganismus kultiviert und dann das Polysaccharidprodukt aus dem Kulturmedium gewinnt.

3. Verfahren nach Anspruch 2, dadurch gekennzeichnet, dass man einen zur Gattung Alteromonas gehörenden Mikroorganismus in einem aus in Meerwasser bzw. künstlichen Meerwasser gelösten Kohlenstoffquellen, Stickstoffquellen, anorganischen Ionen und gegebenenfalls Vitaminen und Aminosäuren bestehenden Medium unter Belüftungsbedingungen bei einer Temperatur von 10°C bis 35°C kultiviert und das gebildete, in der Fermentationsbrühe angereicherte Polysaccharid abtrennt und gewinnt.

4. Verfahren nach Anspruch 3, dadurch gekennzeichnet, dass man einen zur Gattung Alteromonas gehörenden Mikroorganismus bei einer Temperatur von 24°C bis 30°C kultiviert.

5. Verfahren nach Anspruch 3, dadurch gekennzeichnet, dass man die Polysaccharidsubstanz dadurch aus der Fermentationsbrühe gewinnt, dass man diese einer Trennung durch Zentrifugieren unterwirft, der überstehenden Flüssigkeit ein Alkanol zusetzt, durch Abkühlen Ausfällung einleitet und den Niederschlag nach bekannten Methoden gewinnt und reinigt.

6. Verfahren nach einem der Ansprüche 2 bis 5, dadurch gekennzeichnet, dass der Mikroorganismus Alteromonas vaga MP-86 (FERM P-6282) ist.

7. Pharmazeutische Zusammensetzungen, dadurch gekennzeichnet, dass sie aus einer Polysaccharidsubstanz nach Anspruch 1 als Wirkstoff zusammen mit einem pharmazeutisch unbedenklichen Träger bestehen.

8. Polysaccharidsubstanz nach Anspruch 1 zur Verwendung als Arzneimittel.

**Patentansprüche für den Vertragsstaat: AT**

1. Verfahren zur Herstellung einer Polysaccharidsubstanz mit

a) einem Kohlenstoff/Stickstoffverhältnis von 36:1,

b) einem Durchschnittsmolekulargewicht von $5 \cdot 10^4$ bis $15 \cdot 10^4$,

c) der Zuckerzusammensetzung 25±3% Glucose, 25±3% Mannose, 21±2% Galaktose, 14±1% Rhamnose und 5,0±0,5% Fucose,

d) Unlöslichkeit in organischen Lösungsmitteln und Löslichkeit in Wasser,

**0 084 334**

e) positivem Farbentwicklungstest nach der Phenolschwefelsäuremethode und einem Infrarotabsorptionsspektrum wie in Abbildung 3, dadurch gekennzeichnet, dass man einen zur Gattung Alteromonas gehörenden Mikroorganismsus kultiviert und dann das Polysaccharidprodukt aus dem Kulturmedium gewinnt.

2. Verfahren nach Anspruch 1, dadurch gekennzeichnet, dass man einen zur Gattung Alteromonas gehörenden Mikroorganismus in einem aus in Meerwasser bzw. künstlichem Meerwassergelösten Kohlenstoffquellen, Stickstoffquellen, anorganischn Ionen und gegebenenfalls Vitaminen und Aminosäuren bestehenden Medium unter Belüftungsbedingungen bei einer Temperatur von 10°C bis 35°C kultiviert und das gebildete, in der Fermentationsbrühe angereicherte Polysaccharid abtrennt und gewinnt.

3. Verfahren nach Anspruch 2, dadurch gekennzeichnet, dass man einen zur Gattung Alteromonas gehörenden Mikroorganismus bei einer Temperatur von 24°C bis 30°C züchtet.

4. Verfahren nach Anspruch 2, dadurch gekennzeichnet, dass man die Polysaccharidsubstanz dadurch aus der Fermentationsbrühe gewinnt, dass man diese einer Trennung durch Zentrifugieren unterwirft, der überstehenden Flüssigkeit ein Alkanol zusetzt, durch Abkühlen Ausfällung einleitet und den Niederschlag nach bekannten Methoden gewinnt und reinigt.

5. Verfahren nach einem der Ansprüche 1 bis 4, dadurch gekennzeichnet, dass der Mikroorganismus Alteromonas vaga MP-86 (FERM P-6282) ist.

**Revendications pour les Etats Contractants: BE, CH, DE, FR, GB, IT, LI, NL, SE**

1. Substance polysaccharidique ayant:
a) un rapport carbone/azote de 36/1;
b) un poids moléculaire moyen de $5 \times 10^4$ à $15 \times 10^4$;
c) la composition en sucres suivante: 25±3% de glucose, 25±3% de mannose, 21±2% de galactose, 14±1% de rhamnose, 5,0±0,5% de fucose;
d) insolubilité dans les solvants organiques et solubilité dans l'eau;
e) réponse positive à l'essai de développement de couleur par la méthode phénol-acide sulfurique;
f) un spectre d'absorption infrarouge tel que celui qui est présenté sur la Figure 3,
et qui peut être obtenue par culture d'un microorganisme appartenant au genre Alteromonas.

2. Procédé d'obtention d'une substance polysaccharidique ayant:
a) un rapport carbone/azote de 36/1;
b) un poids moléculaire moyen de $5 \times 10^4$ à $15 \times 10^4$;
c) la composition en sucres suivante: 25±3% de glucose, 25±3% de mannose, 21±2% de galactose, 14±1% de rhamnose, 5,0±0,5% de fucose;
d) insolubilité dans les solvants organiques et solubilité dans l'eau;
e) réponse positive à l'essai de développement de couleur par la méthode phénol-acide sulfurique;
f) un spectre d'absorption infrarouge tel que celui qui est présenté sur la Figure 3,
caractérisé en ce qu'il comprend la culture d'un microorganisme appartenant au genre Alteromonas et ensuite la récupération du produit polysaccharidique à partir du milieu de culture.

3. Procédé selon la revendication 2, caractérisé en e que l'on cultive un microorganisme appartenant au genre Alteromonas dans un milieu constitué de sources de carbone, sources d'azote, ions minéraux et, facultativement, vitamines et amino-acides, dissous dans de l'eau de mer, y compris de l'eau de mer artificielle, dans des conditions d'aération, à une température allant de 10°C à 35°C et en ce que l'on sépare et récupère le polysaccharide produit et accumulé dans le bouillon de culture.

4. Procédé selon la revendication 3, caractérisé en ce que l'on cultive un microorganisme appartenant au genre Alteromonas à une température allant de 24°C à 30°C.

5. Procédé selon la revendication 3, caractérisé en ce que l'on récupère la substance polysaccharidique à partir du bouillon de culture en soumettant ce dernier à une séparation centrifuge, puis en ajoutant un alcanol au liquide surnageant, en provoquant la précipitation par refroidissement et en isolant et en purifiant le précipité par des méthodes connues.

6. Procédé selon l'une quelconque des revendications 2 à 5, caractérisé en ce que le microorganisme employé est Alteromonas vaga MP-86 (FERM P-6282).

7. Composition pharmaceutique comprenant comme ingrédient actif une substance polysaccharidique selon la revendication 1 associée à un excipient acceptable du point du vue pharmaceutique.

8. Substance polysaccharidique selon la revendication 1 destinée à être utilisée comme médicament.

**Revendications pour l'Etat Contractant: AT**

1. Procédé d'obtention d'une substance polysaccharidique ayant:
a) un rapport carbone/azote de 36/1;
b) un poids moléculaire moyen de $5 \times 10^4$ à $15 \times 10^4$;
c) la composition en sucres suivante: 25±3% de glucose, 25±3% de mannose, 21±2% de galactose, 14±1% de rhamnose, 5,0±0,5% de fucose;
d) insolubilité dans les solvants organiques et solubilité dans l'eau;

11

e) réponse positive à l'essai de développement de couleur par la méthode phénol-acide sulfurique;

f) un spectre d'absorption infrarouge tel que celui qui est présenté sur la Figure 3,

caractérisé en ce qu'il comprend la culture d'un microorganisme appartenant au genre Alteromonas et ensuite la récupération du produit polysaccharidique à partie du milieu de culture.

2. Procédé selon la revendication 1, caractérisé en ce que l'on cultive un microorganisme appartenant au genre Alteromonas dans un milieu constitué de sources de carbone, de sources d'azote, ions minéraux et, facultativement, vitamines et amino-acides, dissous dans de l'eau de mer, y compris de l'eau de mer artificielle, dans des conditions d'aération, à une température allant de 10°C à 35°C, et en ce que l'on sépare et récupère le polysaccharide produit et accumulé dans le bouillon de culture.

3. Procédé selon la revendication 2, caractérisé en ce que l'on cultive un microorganisme appartenant au genre Alteromonas à une température allant de 24°C à 30°C.

4. Procédé selon la revendication 2, caractérisé en ce que l'on récupère la substance polysaccharidique à partir du bouillon de culture en soumettant ce dernier à une séparation centrifuge, puis en ajoutant un alcanol au liquide surnageant, en provoquant la précipitation par refroidissement et en isolant et en purifiant le précipité par des méthodes connues.

5. Procédé selon l'une quelconque des revendications 1 à 4, caractérisé en ce que le microorganisme employé est Alteromonas vaga MP-86 (FERM-P-6282).

12

FIG.1

FIG.2

FIG. 3